# EUROPEAN PATENT APPLICATION

(11) **EP 2 514 409 A1**
(43) Date of publication of application: **24.10.2012**
(21) Application number: 12164607.9
(22) Date of filing: 18.04.2012
(51) Int. Cl.: A61K 9/00, A61K 31/10, A61K 31/18, A61K 45/06, A61K 47/18, A61K 47/20, A61K 47/32, A61P 29/00

(54) **Topical pharmaceutical compositions of nimesulide and methylsulfonylmethane**

(30) Priority: 19.04.2011 TR 201103770
(71) Applicant: Sanovel Ilaç Sanayi Ve Ticaret Anonim Sirketi, 34460 Sariyer/Istanbul (TR)
(72) Inventor: Cifter, Ümit, 34460 Istanbul (TR); Türkyilmaz, Ali, 34460 Istanbul (TR); Önder, Ramazan, 34460 Istanbul (TR)
(74) Representative: Sevinç, Erkan

(57) **Abstract**

The present invention relates to a topical pharmaceutical composition of nimesulide and methylsulfonylmethane comprising at least one penetration enhancer and gelling agent. Furthermore, the invention relates to process for preparing the said topical pharmaceutical composition and its use for the treatment of pain and inflammatory symptoms associated with muscle-skeletal system, osteoarthritis and rheumatoid arthritis.

## Description

### Field of Invention

The present invention relates to a topical pharmaceutical composition of nimesulide and methylsulfonylmethane comprising at least one penetration enhancer and gelling agent. Furthermore, the invention relates to process for preparing the said topical pharmaceutical composition and its use for the treatment of pain and inflammatory symptoms associated with muscle-skeletal system, osteoarthritis and rheumatoid arthritis.

### Background of Invention

Nimesulide is a non-steroidal anti-inflammatory drug (NSAID) which selectively inhibits the cyclooxygenase-2 (COX-2) enzyme. It has antipyretic, analgesic and anti-inflammatory properties. It is used in osteoarthritis and extraarticular rheumatic diseases, post-traumatic and post-operative inflammations and painful symptoms, high fever and in primary dysmenorrhoea. Its chemical structure is shown with Formula I given below.

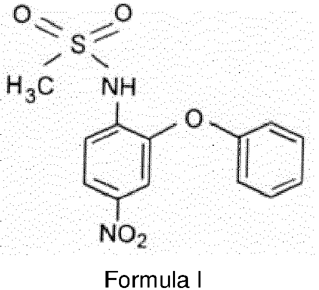

Nimesulide is mostly administrated orally. One disadvantage of the oral administration of nimesulide comprising compositions is that the patient is likely to experience unpleasant side effects, including gastrointestinal irritation. While such irritation may also result in chronic stomach upset, in some cases this can quickly manifest itself in spontaneous gastric bleeding, which can be life threatening.

Thus, the use of nimesulide in treating local pains and inflammations may cause a problem especially for those who have gastrointestinal system disorders. It is possible to develop various locally-administrable topical forms of nimesulide, in order to avoid the systemic side-effects thereof.

On the other hand nimesulide has another disadvantage in percutaneous penetration, it has low penetration and requires relatively longer time periods following the administration thereof. Particularly in acute disorders, there arises the need of enhancing the absorption rate at the site of administration. The skin absorption rate of the relevant product to be used in topical applications, however, is quite significant. Enhancing the absorption rate provides ease of application and increases the molecule's efficiency.

Whilst it is known that surface-active agents and excipients providing percutaneous penetration are used for eliminating the aforesaid problem, particularly the percutaneous penetration enhancers bring about irritations at the primary site of administration.

Methylsulfonylmethane (MSM) is an organosulfur compound and is also known as dimethyl sulfone (DMSO2). It occurs naturally in food in a variety of fruits, vegetables and grains. MSM is commonly used for osteoarthritis, seasonal allergic rhinitis, interstitial cystitis and snoring. MSM is the primary oxidative metabolite product of dimethyl sulfoxide (DMSO).

An additional problem associated with oral pharmaceutical compositions, is that the concentration levels which must be achieved in the bloodstream must be significant in order to effectively treat distal areas of pain and inflammation. These levels are often much higher than would be necessary if it were possible to more accurately target the particular site of pain and injury. Thus there exists a need for a transdermal analgesic formulation which is capable of distal application and which has the ability to alleviate pain and inflammation in a local way.

Also in acute disorders, there arises the need of enhancing the absorption rate at the site of administration. For instance, during which local pains associated with injuries in sportive events are to be urgently alleviated, it becomes necessary to apply local anesthesia to the relevant site.

U.S. Pat. No. 6,444,234 discloses a liquid carrier composition effective for the transdermal delivery of a medicament having a given polarity, said formulation comprising (a) at least one non-aqueous non-toxic solvent; (b) limonene, lemon oil or mixture of limonene and lemon oil; (c) methylsulfonylmethane; (d) a skin stabilizer (e) a solute modifier; and (f) adenosine triphosphate (ATP) or a compound which induces generation of cyclic adenosine 3'5'monophosphate cAMP in situ or cyclic guanosine monophosphate (cGMP) in situ.

In prior art, there are also several patents which disclose NSAIDs with methylsulfonylmethane mostly in oral pharmaceutical dosage forms but none of them selected particularly nimesulide in a topical formulation to combine with methylsulfonlymethane because of its poor transdermal absorption properties.

U.S. Pat. No. 6,416,772 discloses a liquid composition applied transdermally for relief of pain comprising alcohol, glycerin and an analgesic agent; the analgesic agent comprising a derivative of salicylic acid, methylsulfonylmethane and emu oil. But it is silent about the penetration problems and the use of dimethyl sulfoxide and gelling agents in combination with methylsulfonylmethane and nimesulide.

The compositions described above have at least one disadvantage in that the amount of drug delivered transdermally is not maximized. Accordingly, there exists need for a percutaneous delivery system for the drug nimesulide and methylsulfonylmethane which optimizes the delivery of them through the skin.

On the other hand, commercially-available products containing nimesulide form yellow spots at the administration site and similarly form skin stains. These facts are because of the structural features of active ingredient nimesulide and the products in which the active agent is present cause aesthetically-undesired color formations following its administration.

It is therefore an object of the present invention to provide a topical pharmaceutical composition of nimesulide and methylsulfonylmethane which is more effective for purposes of percutaneous delivery of them through the skin and preventing possible stain and colour problems which occurs at the site of administration.

### Summary of the Invention

The present invention relates to an easily applicable nimesulide and methylsulfonylmethane topical pharmaceutical composition, which overcomes the above described problems in prior art and have additive advantages over them.

Accordingly, the main object of the present invention is to increase the rate of percutaneous penetration, thereby shortening the time period in which the active agents exert their effect. The rate of percutaneous penetration of said combination is enhanced with the dimethyl sulfoxide and gelling agents it contains. Also triethanolamine has a synergistic effect over this penetration enhancing activity of them.

Another object of the present invention is to obtain a stable topical pharmaceutical composition of nimesulide and methylsulfonylmethane during the shelf-life and to exhibit high safety when applied to skin. Also surface active agents helps the formulaiton to be stable over the shelf life.

A further object of the present invention is to obtain a formulation with local anesthetic effect, with the menthol used in said combination stimulating the receptors by which the cold sensation is perceived.

Yet a further object of the present invention is to obtain a product, prevents the possible stain problem which occurs at the site of administration.

Accordingly, a topical pharmaceutical composition, more specifically a gel composition has been developed to achieve all objects referred above.

In a preferred embodiment according to the present invention, said novelty is realized with nimesulide and methylsulfonylmethane comprising at least one penetration enhancer and gelling agent.

According to the preferred embodiment, the penetration enhancer is dimethyl sulfoxide.

In another preferred embodiment according to the present invention, the gelling agents are selected from the group comprising carbomer, carbomer copolymers, hydroxypropyl cellulose, hydroxyethyl cellulose, hydroxypropyl methyl cellulose, methyl cellulose, carboxymethyl cellulose, gelatin, aluminum monostearat, dextrin, sodium alginate, pectin, carrageanen, xanthan or mixtures thereof. Preferably the gelling agents are carbomer and carbomer copolymers.

According to a preferred embodiment of the present invention, the topical pharmaceutical composition further comprises triethanolamine.

According to the preferred embodiment, the weight ratio of triethanolamine and carbomer is from 10:1 to 1:10 by weight, preferably it is from 5:1 to 1:5 by weight, more preferably it is from 2:1 to 1:2 by weight of the total composition.

According to the preferred embodiment of the present invention the amount of dimethyl sulfoxide is from 0.5 to 30.0% by weight of the total composition, preferably it is 5.0 to 25.0% by weight of the total composition.

According to a preferred embodiment of the present invention, the topical pharmaceutical composition, further comprises surface active agents which are selected from the group comprising polysorbate, glyceryl monostearate, polyethylene glycol succinate, oleic acid, dietanolamin, sodium lauryl sulfate, propylene glycol or mixtures thereof; preferably the surface active agent is polysorbate.

According to the preferred embodiment of the present invention the amount of polysorbate is 0.05 to 10.0% by weight of the total composition, preferably it is 0.10 to 5.0% by weight of the total composition.

According to a preferred embodiment of the present invention, the topical pharmaceutical composition further comprises menthol, wherein the amount of menthol is between 0.10 to 15.0% by weight of the total composition, preferably it is 1.0 to 10.0% by weight of the total composition.

According to a preferred embodiment of the present invention, the topical pharmaceutical composition further comprises viscosity enhancers, dissolving solvents, chelating enhancers or mixtures thereof.

According to a preferred embodiment of the present invention, the topical pharmaceutical composition is in the form of gel, ointment, cream, spray or lotion, preferably it is in the form of gel.

In a further preferred embodiment of the present invention, said topical pharmaceutical composition comprise the following;

| | | |
|---|---|---|
| a. | nimesulide | 0.50 to 15.0 % by weight |
| b. | methylsulfonylmethane | 1.0 to 20.0 % by weight |
| c. | carbomer | 0.10 to 5.0 % by weight |
| d. | triethanolamine | 0.10 to 5.0 % by weight |
| e. | dimethyl sulfoxide | 0.5 to 30.0 % by weight |
| f. | disodium EDTA | 0.001 to 0.50% by weight |
| g. | polyethylene glycol | 1.0 to 50.0 % by weight |
| h. | menthol | 0.10 to 15.0 % by weight |
| i. | polysorbate | 0.05 to 10.0 % by weight |
| j. | glycerin | 1.0 to 50.0 % by weight |
| k. | ethyl alcohol | 1.0 to 50.0 % by weight |
| I. | purified water | 1.0 to 60.0 % by weight |

Another embodiment of the present invention provides a method for preparing the topical pharmaceutical composition according to the present invention and this method comprising the steps of;
a. adding disodium EDTA into purified water, then adding carbomer therein and stirring the resultant mixture so as to swell the later and to obtain the first mixture;
b. adding and dissolving nimesulide into polyethylene glycol in a separate container, then adding and dissolving methylsulfonylmethane into this mixture, then adding dimethyl sulfoxide into and stirring it in the resultant mixture so as to obtain the second mixture;
c. adding glycerin and menthol previously dissolved in ethyl alcohol into the second mixture, then adding polysorbate into the latter and stirring the resultant mixture;
d. adding the second mixture into the first mixture under stirring; and
e. adding triethanolamine into the resultant final mixture, then gellifying and stirring this mixture with adding purified water into it.

According to another preferred embodiment of the present invention, the topical pharmaceutical composition is used in the treatment of pain and inflammatory symptoms associated with muscle-skeletal system, osteoarthritis and rheumatoid arthritis.

Further advantages and embodiments of the present invention will become apparent from the following description

### Detailed Description of Invention

According to the present invention, a novel formulation with anti-inflammatory and analgesic activities is obtained, which surprisingly is rapidly absorbed and gives local anesthetic effect without irritating the skin and avoiding the possible staining problems.

The topical pharmaceutical compositions of the invention comprise from 0.5 to 15.0% nimesulide, preferably from 1.0 to 10.0% by weight of the total composition and comprise from 1.0 to 20.0% methylsulfonylmethane, preferably from 5.0 to 15.0% by weight of the total composition.

The topical pharmaceutical compositions of the invention comprise from 0.5 to 30.0% dimethyl sulfoxide, preferably from 5.0 to 25.0%, more preferably from 10.0 to 20.0% by weight of the total composition. Dimethyl sulfoxide helps the composition of the present invention to improve and enhance the penetrating and spreading properties through the skin. It also helps to carry out other components easily and without damaging the membranes into biological system. These properties, surprisingly is found to have synergistic effect over nimesulide and methylsulfonlymethane topical composition to have better percutaneous penetration. Accordingly, dimethyl sulfoxide is used as a topical analgesic, a vehicle for topical application of pharmaceuticals, as an anti-inflammatory and an antioxidant. Other suitable penetration enhancers which can be used for the composition of the present invention may comprise 1,3-didocyl urea, 1,3-difenyl urea, 3-caren, 7-oxabicylo 2,2-heptan, ascaridol, dimetyl isosorbide, dimetyl formamide (DMF), d-Limonen, isopropyl myristat, carveol, carvon, menton, N-metyl-2-pyrolidon, NN-dimetyl toluamide, oleic acid, pinen oxide, puiegon, scylohexan oxide, siklopenten oxide, sodyum lauryl sulfate, terpinen-4-oi urea, a-pinen, a-terpineol or mixtures thereof.

The topical pharmaceutical compositions of the invention comprise from 0.01 to 10.0% gelling agents, preferably from 0.05 to 5.0% by weight. Suitable gelling agents may comprise but not limited to carbomer, carbomer copolymers, hydroxypropyl cellulose, hydroxyethyl cellulose, hydroxypropyl methyl cellulose, methyl cellulose, carboxymethyl cellulose, gelatin, aluminum monostearat, dextrin, sodium alginate, pectin, carrageanen, xanthan or mixtures thereof. Preferably the gelling agents are carbomer and carbomer copolymers.

Furthermore, the topical pharmaceutical compositions of the invention comprise triethanolamine as emulsifying agent. In the present formulation triethanolamine enhance the viscosity and helps gellifying of the formulation by the help of pH adjusters. On the other hand, with adjusting the amount of triethanolamine, the color of the composition is controlled and possible skin stains are prevented.

Surprisingly it is found that when the weight ratio of triethanolamine to carbomer is from 10:1 to 1:10 by weight, preferably from 5:1 to 1:5 by weight, more preferably from 2:1 to 1:2 by weight; it enhance the penetration properties of the formulation in combination with dimethyl sulfoxide. Accordingly, triethanolamine and carbomer helps to obtain the desired viscosity in a stable level to enhance the penetration of the topical composition and its stabilizer and emulsifier property is also help them to show this effect easily.

Suitable surface active agents may comprise but not limited to polysorbate, glyceryl monostearat, polyethylene glycol succinate, oleic acid, diethanolamine, sodium lauril sulfate, propylene glycol or mixtures thereof. Preferably the surface active agent is polysorbate 20, polysorbate 40, polysorbate 60, polysorbate 80 or mixtures thereof. The most preferred one is polysorbate 80. The amount of polysorbate 80 is from 0.05 to 10.0%, preferably 0.1 to 5.0% by weight of the total composition. Polysorbate has also a stabilisator effect when it is used in these amounts and helps the formulation to be stable over the shelf life.

Furthermore, the topical pharmaceutical compositions of the invention comprise menthol from 0.10 to 15.0%, preferably from 1.0 to 10.0% by weight of the total composition. Menthol used in the formulation gives anesthetic effect at the side of administration as a result of stimulating the receptors by which cold sensation is perceived.

Another advantage of menthol is to provide significant relief of pain associated with mild to moderate muscle strain in adult patients. Also menthol helps to mask the bad odour of the active ingredients and other excipients used in the formulation to obtain a good patient compliance when applying to skin.

The pharmaceutical compositions according to the present invention may also comprise one or more pharmaceutically acceptable excipients. Such proper pharmaceutically acceptable excipients comprise, but are not limited to viscosity enhancers, dissolving solvents, chelating enhancers or mixtures thereof.

Suitable viscosity enhancers may comprise but not limited to glycerin, pullulan, dextran, cellulose and derivatives, chitosan or mixtures thereof. Preferably the viscosity enhancer is glycerin and the amount is from 1.0 to 50.0%, more preferably from 5.0 to 30.0% by weight of the total composition. These amounts of glycerin improve the spreading properties and minimize any balling up or drying of the compositions of the present invention when it is rubbed on the skin.

Suitable dissolving solvents may comprise but not limited to ethyl alcohol, polyethylene glycol, glycerin, isopropyl alcohol and purified water. Preferably ethyl alcohol, polyethylene glycol and purified water are used. Ethyl alcohol is also utilized as a microbiological preservative.

Suitable chelating agents may comprise but not limited to disodium EDTA, calcium EDTA, and/or EDTA. The most preferred one is disodium EDTA.

The pharmaceutical compositions according to the present invention provides spreadable, semi-solid and jelly-like gel compositions of nimesulide and methylsulfonylmethane. However, the topical compositions of the invention may also take the form of ointment, cream, spray or lotion.

Accordingly, the present invention may be used for treating pain and inflammatory symptoms associated with muscle-skeletal system, osteoarthritis and rheumatoid arthritis.

This invention is further defined by reference to the following example. Although the example is not intended to limit the scope of the present invention, it should be considered in the light of the description detailed above. It will be apparent to those skilled in the art that many modifications, both to materials and methods, may be practiced without departing from the scope of the invention.

### Example

| **Content** | **amount (%) (w/w)** |
|---|---|
| nimesulide | 1.0 % |
| methylsulfonylmethane | 5.0 % |
| carbomer | 1.0 % |
| triethanolamine | 0.20 % |
| dimethyl sulfoxide | 6.0 % |
| disodium EDTA | 0.10 % |
| polyethylene glycol | 20.0 % |
| menthol | 2.50 % |
| polysorbate | 2.0 % |
| glycerin | 10.0 % |
| ethyl alcohol | 10.0 % |
| purified water | 42.2 % |

Disodium EDTA is added into purified water, then carbomer is added therein under stirring so as to swell the resultant mixture for about 60 min. and it is homogenized to obtain the first mixture. Nimesulide is dissolved in polyethylene glycol in a separate container, then dissolving methylsulfonylmethane is added and dissolved in this mixture. Then dimethyl sulfoxide is added into and stirring it in the resultant mixture so as to obtain the second mixture. Glycerin and menthol is previously dissolved in ethyl alcohol and added into the second mixture, then polysorbate is added into the latter and the resultant mixture is stirred.

The second mixture is added to the first mixture to obtain the final mixture under stirring for about 10 min. Triethanolamine is added into the resultant final mixture, then, it is brought into a gelled state by adding purified water under stirring.

## Claims

1. A topical pharmaceutical composition of nimesulide and methylsulfonylmethane comprising at least one penetration enhancer and gelling agent wherein the penetration enhancer is dimethyl sulfoxide.

2. The topical pharmaceutical composition according to claim 1, wherein the gelling agents are selected from the group comprising carbomer, carbomer copolymers, hydroxypropyl cellulose, hydroxyethyl cellulose, hydroxypropyl methyl cellulose, methyl cellulose, carboxymethyl cellulose, gelatin, aluminum monostearat, dextrin, sodium alginate, pectin, carrageanen, xanthan or mixtures thereof.

3. The topical pharmaceutical composition according to claim 3, wherein the gelling agents are preferably carbomer and carbomer copolymers.

4. The topical pharmaceutical composition according to claim 1, further comprising triethanolamine.

5. The topical pharmaceutical composition according to claims 4 and 5, wherein the weight ratio of triethanolamine to carbomer is from 10:1 to 1:10 by weight, preferably it is from 5:1 to 1:5 by weight, more preferably 2:1 to 1:2 by weight of the total composition.

6. The topical pharmaceutical composition according to claims 1 and 2, wherein the amount of dimethyl sulfoxide is from 0.50 to 30.0 % by weight of the total composition, preferably it is 5.0 to 25.0 % by weight of the total composition.

7. The topical pharmaceutical composition according to claim 1, further comprising surface active agents.

8. The topical pharmaceutical composition according to claim 8, wherein the surface active agent is selected form the group comprising, polysorbate, glyceryl monostearate, polyethylene glycol succinate, oleic acid, diethanolamin, sodium lauryl sulfate, propylene glycol or mixtures thereof; preferably the surface active agent is polysorbate.

9. The topical pharmaceutical composition according to claim 9, wherein the amount of polysorbate is 0.05 to 10.0 % by weight of the total composition, preferably it is 0.10 to 5.0 % by weight of the total composition.

10. The topical pharmaceutical composition according to claim 1, further comprising menthol.

11. The topical pharmaceutical composition according to claim 11, wherein the amount of menthol is between 0.10 to 15.0 % by weight of the total composition, preferably it is 1.0 to 10.0 % by weight of the total composition.

12. The topical pharmaceutical composition according to any of the preceding claims, further comprising viscosity enhancers, dissolving solvents, chelating enhancers or mixtures thereof.

13. The topical pharmaceutical composition according to any of the preceding claims, wherein it is in the form of gel, ointment, cream, spray or lotion, preferably it is in the form of gel.

14. The topical pharmaceutical composition according to any of the preceding claims,
comprising,
| | | |
|---|---|---|
| a. | nimesulide | 0.50 to 15.0 % by weight |
| b. | methylsulfonylmethane | 1.0 to 20.0 % by weight |
| c. | carbomer | 0.10 to 5.0 % by weight |
| d. | triethanolamine | 0.10 to 5.0 % by weight |
| e. | dimethyl sulfoxide | 0.5 to 30.0 % by weight |
| f. | disodium EDTA | 0.001 to 0.50% by weight |
| g. | polyethylene glycol | 1.0 to 50.0 % by weight |
| h. | menthol | 0.10 to 15.0 % by weight |
| i. | polysorbate | 0.05 to 10.0 % by weight |
| j. | glycerin | 1.0 to 50.0 % by weight |
| k. | ethyl alcohol | 1.0 to 50.0 % by weight |
| I. | purified water | 1.0 to 60.0 % by weight |

15. Process for preparing the topical pharmaceutical composition according to any of the preceding claims, comprising the steps of;
a. adding disodium EDTA into purified water, then adding carbomer therein and stirring the resultant mixture so as to swell the later and to obtain the first mixture;
b. adding and dissolving nimesulide into polyethylene glycol in a separate container, then adding and dissolving methylsulfonylmethane into this mixture, then adding dimethyl sulfoxide into and stirring it in the resultant mixture so as to obtain the second mixture;
c. adding glycerin and menthol previously dissolved in ethyl alcohol into the second mixture, then adding polysorbate into the latter and stirring the resultant mixture;
d. adding the second mixture into the first mixture under stirring; and
e. adding triethanolamine into the resultant final mixture, then gellifying and stirring this mixture with adding purified water into it.

16. The topical pharmaceutical composition according to any previous claims, for use in the treatment of pain and inflammatory symptoms associated with muscle-skeletal system, osteoarthritis and rheumatoid arthritis.
